Europäisches Patentamt

European Patent Office

Office européen des brevets

(11) Publication number: **0 476 556 A2**

# EUROPEAN PATENT APPLICATION

(21) Application number: **91115648.7**

(22) Date of filing: **14.09.91**

(51) Int. Cl.5: **G01N 21/76, G01N 33/53**

(30) Priority: **18.09.90 JP 249879/90**

(43) Date of publication of application:
**25.03.92 Bulletin 92/13**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Motsenbocker, Marvin A.**
**302, 32-3, Tsukaguchicho 5-chome**
**Amagasaki, Hyogo 661(JP)**
Inventor: **Shintani, Motoaki**
**2-64, Seiwadainishi 5-chome**
**Kawanishi, Hyogo 666-01(JP)**
Inventor: **Kondo, Koichi**
**6-3, Kabutodai 3-chome, Kizu-cho**
**Sohraku-gun, Kyoto 619-02(JP)**
Inventor: **Masuya, Hirotomo**
**3-25, Fushimidai 3-chome, Inagawa-cho**
**Kawabe-gun, Hyogo 666-02(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Method for determination of a light sensitive substance.**

(57) There is disclosed a method for determination of a light sensitive substance wherein irradiation of lumigenic substance-light sensitive substance solution with modulated light is used to generate short wavelength light proportionally to the concentration of the light sensitive substance. Synchronous detection allows high sensitivity. The method provides for diagnostic assays of greater simplicity and convenience.

EP 0 476 556 A2

## FIELD OF THE INVENTION

The present invention relates to a method for quantitation of a light sensitive substance. More particularly, it relates to chemiluminescence detection of water borne analytes, specifically, to a chemiluminescence detection technique employed in diagnostic procedures.

## BACKGROUND OF THE INVENTION

Chemistry based determination procedures are extremely useful for detecting and quantitating various substances of medical, environmental and industrial importance which appear in liquid medium at very low concentrations. Unless the analyte is itself strongly fluorescent or chemiluminescent, it cannot be directly detected at low concentration. Thus, the presence of analyte is measured indirectly by use of a signal generating molecule, i.e., chemical that makes a signal (usually a catalyst) in a chemical detection step. Preceding this detection step are other chemical reactions, which usually include binding reactions (i.e., chemical reactions in which different molecules bind very tightly to each other) of antigens, antibodies or nucleic acids. The catalyst used for detection is most commonly an enzyme. These enzymatic detection reactions are characterized as having specific incubation (pH, salt, temperature, substrate, buffer composition, etc.) requirements to which the enzyme is exposed for a period of time after which the product of the enzyme reaction is measured. Because of the inherent instability of enzymes, diagnostic assays which incorporate an enzyme catalyst tend to have a shorter storage life and require more stringent detection reaction conditions than do assays that do not employ an enzyme catalyst.

Many advances in diagnostics in recent years have resulted from improvements in the detection step of assays. Two of the most important advances which overcome the background problems of fluorescence are the time resolved fluorescence [Soini and Kojiola, Clin. Chem., 29, 65-68 (1983)] and chemiluminescence [Schroeder et al., Methods in Enzymology, 57, 424-459 (1978)] techniques.

In the time resolved fluorescence technique, the long electronic decay time of a transition atom is used to remove stokes shift fluorescence (longer light wavelength fluorescence from shorter wavelength excitation) by letting the stokes shift fluorescence decay before measuring light emission from the transition metal atom. One problem with the time resolved fluorescence technique is that transition atoms having suitable electronic decay rates are likely to be quenched by water and must be protected, usually by addition of a micelle-creating substance. This complicates use of the transition atom by necessitating an extra step in the detection procedure. A second problem is that the transition atom absorbs light poorly and must exist as a complex with another compound that can absorb exciting light and then transfer the absorbed energy to the transition atom. The atom is typically chelated to a light absorber for this purpose. Another limitation is that biological samples such as blood or urine frequently contain significant concentrations of transition state atoms and thus suffer a background. Yet another limitation to the time resolved fluorescence method is that an expensive or complicated short wavelength light source is needed in conjunction with narrow optical filtering to reduce background. Use of narrow optic filters greatly decreases light throughput of the system and lowers sensitivity. Yet another disadvantage of the time resolved fluorescence technique is after the excitation light is turned off, a time period must elapse between removal of the excitation light and beginning the light measurement in order to eliminate fluorescence background and part of the after-glow of the flash lamp used in the excitation. Because of the delay time some of the desired signal is discarded. The after-glow is the persistent emission of light from a hot light source after it has been turned off and it directly limits sensitivity by causing a high background. Unfortunately, all pulsed white light sources suffer this glow and it is desired to use a pulsed light which does not create this background.

On the other hand, chemiluminescence has become a popular detection technique because of the high sensitivity attainable with photon counting in the absence of a background. Three types of chemiluminescence reactions have become most popular. In one, chemilumigenic derivatives (i.e, chemicals that produce light) are used, for example, to label an antibody. These are oxidized by reaction with an oxidant such as hydrogen peroxide during the detection step to produce light photons directly proportional to the antibodies present in the readout reaction. In this system, typically about 1% of the labels used (e.g., luminol derivative or acridinium ester derivative) give rise to a photon. Diagnostic assay kits which employ this first type of chemiluminescence suffer the disadvantage of requiring the storage and use of an oxidant which is needed for generation of chemiluminescence of the chemilumigenic labeled compound. Another disadvantage is that measurements must be done within a short period of time. The chemilumigenic reaction is sudden and light measurements cannot be repeated because the chemilumigenic labeled compound is destroyed during a short time. In a second type of chemiluminescence detection reaction, for example, the antibody is labeled with an enzyme that interacts with an oxidant such as hydrogen peroxide or perborate and a chemilumigenic compound such as luminol to generate light [Baret and Fert, J. Biolum. Chemilum., 4, 149-153 (1989)]. The principal advantages of these systems are that much higher light levels are involved and detection of chemiluminescence can be made over long time periods. The disadvantages of these systems include problems from using an enzyme label and from the need to add an oxidant such as hydrogen peroxide to the detection solution. The biggest drawback to these chemiluminescence techniques is the high background light caused by including a strong oxidant such as hydrogen peroxide at high concentration (typically 1 mM) in the incubation solution. Any method which eliminates need for a high oxidant concentration would be an advance to the art.

A third type of system overcomes the disadvantage of needing to store and mix an unstable oxidant with the detection solution by the use of a stable dioxetane compound as an enzymatic substrate [Bronstein et al., J. Biolum. Chemilum., 4, 99-111 (1989)]. Although the stability and state of the art sensitivity of these labeled dioxetane compounds are a big improvement to the art, the chemiluminescence efficiency of this system is inherently poor and special enhancers, i.e., chemicals which improve the chemical reaction, need to be combined with the substrate solution to obtain good light output. Also, the disadvantages of using an enzyme label remain.

As described hereinabove, one focus in the development of chemiluminescence systems has been in chemistries that do not require addition of an oxidant. A number of strategies have been used, such as using heat to develop the chemiluminescence readout signal [Hummelen et al., Pure Appl. Chemistry, 59, 639-644 (1987)]. A successful strategy has been to develop chemilumigenic enzyme substrates that already contain a high energy but stable dioxetane as described above under the third type of system described above. A variety of alternative chemiluminescence oxidation systems are possible but have not been put to practical use for various reasons. One such example that has been used for many years and which does not require hydrogen peroxide is photoactive dye (chemical that is sensitive to light) sensitized luminol chemiluminescence [Matheson and Lee, Photochem. Photobiol., 24, 605-616 (1976)]. In this system, light irradiation generates singlet oxygen and/or a light activated dye reactant which then reacts with luminol to generate chemiluminescence with good quantum efficiency. A variation on this technique is to improve sensitivity by building up a high concentration of reactive intermediates and then using heat to develop a light flash (European Patent Application 89110383.0, U.S. Patent Application Ser. No. 204,055). This system is inconvenient however because of the extra complexity needed to develop the light flash.

A common problem present in this and in all light irradiation techniques is that irradiating a sample at one wavelength causes a large background light signal at a longer wavelength due to stokes shift fluorescence. Such background fluorescence severely limits sensitivity of fluorescence techniques. In fact, both the fluorescence and chemiluminescence techniques are popular but the principal advantage of using chemiluminescence over fluorescence is that chemiluminescence detection does not suffer the stokes fluorescence background drawback.

OBJECTS OF THE INVENTION

The present inventors made intensive studies on photoactive dyes to increase the sensitivity of enzyme catalyzed luminol chemiluminescence assays and they made the surprising discovery that adding trace amounts (less than 1 ng/ml) of photoactive dye to luminol solutions under certain conditions generated as much light as the enzyme catalyst being quantitated. Upon further optimization it was discovered that conventional limitations to the light irradiation technique (stokes fluorescence) could be overcome by careful selection of a dye catalyst having a suitable long wavelength absorbance and the use of deep red light from an inexpensive light source that lacks the after-glow effect for stimulation of the catalyst. Furthermore, the technique of modulating the irradiation light can be used with synchronous (detecting signals in the same periodic manner as the light is varied) detection of the emitted light to eliminate the constant part of the light signal and allow use of wider filters. This allowed improvements in sensitivity by decreasing the unwanted background signal while maintaining a strong light signal.

One object of the present invention is to improve sensitivity of the assay detection system and to eliminate the need for a high energy compound such as hydrogen peroxide to generate chemiluminescence from a chemilumigenic substance such as luminol in sensitive assay reactions. This is made possible by a method that generates the oxidant via a catalyst during the reaction itself. In this method red light supplies energy to a photosensitive substance which catalytically reacts with molecular oxygen to generate an oxidant. The oxidant (singlet oxygen or activated dye reactant or both) reacts directly with luminol to generate blue light, obviating the need to prepare or store another oxidant such as hydrogen peroxide or perborate for this purpose.

Sensitivity is improved in two ways. Because long wavelength light (e.g., 670nm) is used to generate shorter (e.g., 425nm) wavelength light, the stokes shift is eliminated and there is no background fluorescence to limit sensitivity. Because the reaction is directly controlled by the laser, other novel means are available to control the background and thereby improve sensitivity. For example, non-specific undesired background chemiluminescence of luminol and background (room) light occurs at a constant rate but the specific chemical reaction can be repeatedly turned on and off rapidly. By modulating the amplitude of the light driven reaction and using frequency selection to measure the resulting emitted light, the contribution to the output light from the background is removed. The light itself can be controlled, for example by varying the input power to a light emitting diode or laser diode, or the light beam can be interrupted by a chopper (mechanical) mechanism. In one preferred embodiment, the laser amplitude is modulated at a 500Hz rate and a lock-in amplifier is used to remove the constant background signal from the 500Hz dye catalyzed chemiluminescence signal. In another preferred embodiment time gating of excited light and of the light measurement is used to obtain a measurement in the presence of irradiation light.

Another object of the invention is to decrease the cost and complexity of the instrument used for detection of the catalyst. This is achieved by allowing use of an inexpensive red laser light source and use of a pulse or modulation technique to eliminate the background signal.

Yet another object of the invention is to allow use of wider band pass filters with the photon-counting technique. Use of wider bandpass filters allows more light to enter the light detector. This is made possible by the use of long-wave length light to excite the dye catalyst and a short wavelength sensitive photonmultiplier tube to measure emitted light. This is also made possible by the use of the pulse technique itself. By pulsing the dye excitation light and measuring the PMT (Photomultiplier tube) output at some time (typically 10 to 100 microseconds) after extiguishing the light source, the transient overloading of the PMT which gives rise to a dark current background can be partly overcome.

These objects as well as other objects and advantages of the present invention will become apparent to those skilled in the art from the following description with reference to the accompanying drawings.

## BRIEF EXPLANATION OF THE DRAWINGS

Fig. 1 is a diagram illustrating the chemiluminescence instrument of the present invention using lock-in amplifier.

Fig. 2 is a graph illustrating the methylene blue standard curve comparing lock-in detection with unprocessed signal detection.

Fig. 3 is a diagram illustrating the chemiluminescence instrument of the present invention using photon counting.

Fig. 4 is a graph illustrating a methylene blue standard curve (photons counted during laser off-time).

Fig. 5 is a graph illustrating a methylene blue standard curve (photons counted at all times).

## SUMMARY OF THE INVENTION

According to the present invention, there are provided:

(1) A method for determination of a light sensitive substance comprising irradiating the light sensitive substance in the presence of a lumigenic substance with modulated light, detecting shorter wavelength emitted light proportional to the concentration of the light sensitive substance and synchronously detecting emitted light; and

(2) A method for determination of a light sensitive substance comprising pulse-irradiating the light sensitive substance in the presence of a lumigenic substance and detecting shorter wavelength emitted light in the absence of the pulsed excitation light proportional to the concentration of the light sensitive substance.

## DETAILED DESCRIPTION OF THE INVENTION

The light sensitive substances used in the present invention include a photoactive dye. The photoactive dye is defined as a catalytic substance which upon light absorption becomes activated and generates chemiluminescence of a lumigenic substance by either or both photooxygenation Type I or Type II processes [Foote, Science, 162, 963-970 (1968)]. It is desired that the dye has a significant optical absorption band longer than 550nm wavelength and preferably longer than 650nm wavelength. The following dyes and the derivatives have one or more absorption bands in this region and were found to work well in catalyzing the chemiluminescence of the lumigenic substance: xanthine, eosin, porphyrin, methylene blue, rose bengal, chlorin, thionine and their derivatives. Any red light photosensitive material may have utility in the invention. For example, many photoactive substances have been evaluated for photodynamic treatment of cancer and these can be used [Andersson-Engels et al., Anal. Chem., 61, 1367A-1373A (1989)].

In the quantitative analytical technique of the present invention, during the detection step, the light sensitive substance is quantitated by incubating it in a solution of chemilumiphore and buffer. The chemilumiphore is a molecule that generates light upon interaction with photoactive dye or an oxidant generated by photoactive dye. Any lumigenic substance can be used in the present invention. A lumigenic substance having a light emission maximum less than 550 nm is preferred and examples thereof include luminol, lucigenin, luciferin, isoluminol, benzopyrene and the like. Derivatives of these substances can also be used. Luminol is most preferred. The lumigenic substances such as luminol can generally be used at any concentration of at least 0.01 mM and preferably between 0.1 mM and 2 mM. For the best detection sensitivity, the luminol is recrystallized from base. Although the buffer composition is not critical, the pH is preferably above pH 10 and most preferably between pH 12 and pH 13. The temperature of the reaction is also not critical and a temperature of between 15 and 35°C is preferred.

Red light of wavelength longer than 550 nm is preferred and light of wavelength longer than 650 nm is most preferred for excitation of the catalyst. For light detection, photon counting is required to obtain the best sensitivity, although other detectors such as photodiodes or photo-FETs can be used. If necessary, an optic filter between the chemistry and the detector can be included to prevent excessive excitation light from entering the photo-detector. To improve measurement precision, repeated measurements for time periods of up to about 10 minutes are preferred.

The background light signal is constant and is caused by non-specific oxidation of luminol. This background is the principal limitation to detection sensitivity and a principal aim is to diminish it. The technique of synchronous detection or pulse gated detection can be used to remove the background signal from the total signal and thus improve sensitivity. In the former technique, the light intensity is varied and the resulting signal is processed to remove the non-varying (background) unwanted component. In the latter technique, there is no light scattering background. Many diode light sources are easily controlled (e.g., laser diode, emitting diode) by controlling their input power to achieve modulation of the light. Other means can be employed such as for example use of a chopper to interrupt the light beam. Use of a suitable optic filter between the excitation light source and the chemistry is preferred for broadband light sources in order to prevent unwanted light from entering the photo-detector. A diode laser is preferred for excitation because its light output intensity can be easily modulated and the light is monochromatic.

In the synchronous detection technique, the excitation light is periodically modulated and the detected signal is demodulated to extract the part due to red light activation. Use of a lock-in amplifier is preferred for the demodulation step, although the separation of background from the total signal can be performed by computer during or after data collection.

In the pulse gated detection technique, the light signal is measured during a time interval following red light irradiation of the chemistry reaction but before substantial completion of the reaction. The actual laser on and laser off time periods are dependent on the chemistry reaction time course and are ideally shorter than the reaction half life time period. A time period of between 1 microsecond and 100 milliseconds is suitable and between 10 microseconds and 10 milliseconds is especially suitable. It is preferred to use light on and light off intervals of approximately equal duration, although the light on time period can be up to 10 times shorter than the time off period, if a bright enough laser light source is used. For luminol reaction with photosensitive dye in basic solution, the time period of between 200 microseconds and 2 milliseconds is especially suitable. Further improvement in sensitivity can be obtained under strong light conditions by waiting a short time after extinguishing the light and beginning the photon measurement. A delay period of from 1 microsecond to 10 milliseconds is desirable and the optimum time period is chosen empirically while using maximum light conditions. The measurement is performed repeatedly and the photon pulses are summed. The total measurement time may range from 1 sec to as much as 10 minutes.

According to the present invention, for example, it is possible to detect and quantitated various substances of medical, environmental and industrial importance which appear in liquid medium at very low concentrations. Among them, in particular, there are substances which are utilized in clinical tests. Examples thereof include proteins and hormones contained in body fluids such as human immunogloblin G, human immunogloblin M, human immunogloblin A, human immunoglobulin E, human albumin, human fibrinogen (fibrin and its degradation products) $\alpha$-fetoprotein, C-reactive protein, $\beta_2$-microglobulin, myoglobin, carcinoembryonic antigen, hepatitis virus antigen, human chorionic gonadotropin, human placental lactogen, insulin and the like as well as drugs.

The body fluids described above include blood such as whole blood, serum, plasma and the like, urine, marrow liquid, extract from tissue and the like.

In the assay method, when a light sensitive substance having a substantial optical absorption band longer than 650 nm wavelength, for example, a derivative of methylene blue is used, the methylene blue derivative can be chemically bound to hapten, antigen or antibodies against them to prepare a dye catalyzed label.

Examples of assay reagents using such a dye label include the following kit for a sandwich technique:

(1) An antibody immobilized on a solid phase;

(2) The dye catalyst conjugated to a second antibody;

(3) A standard sample of an analyte molecule;

(4) A buffer solution used for dilution of the above reagents of (2) and (3) and a sample to be tested (Any buffer which can be used for dilution of these reagents and sample can be used. Examples thereof include phosphate buffer and glycine buffer of pH 6 to 9.);

(5) A buffer solution used for washing the solid phase after incubation (Any buffer which can be used for washing the support can be used. Examples thereof include phosphate buffer and glycine buffer.); and

(6) Reagents necessary for the assay of the dye catalyzed activity. Examples thereof include luminol dissolved in an alkaline solvent.

The above kit can be used, for example, as follows:

A solution of the standard sample of the analyte molecule or a sample to be tested (about 10 to 200 $\mu$l) is diluted with the reagent (4), a certain amount of the reagent (1) and then the reagent (2) (10 to 800 $\mu$l) are added to the solution, and the mixture is reacted at about 0 to 40°C. After the reaction for about 10 minutes to 2 days, the reaction mixture is washed with the reagent (5) and the catalytic activity of the dye bound to the solid phase is measured. That is, the chemical reaction solution is added, and the mixture is immediately subjected to the chemiluminescence measurement system of the present invention to measure the amount of chemiluminescence in the reaction mixture.

According to the present invention, a high sensitivity measurement is made possible with a simple operation.

According to the present invention, determination of a light sensitive substance can be carried out without using any high energy compound such as hydrogen peroxide, and the sensitivity of an instrument used for the detection can be improved. In addition, the present invention allows reduction of instrument cost and simplification of its structure.

The following Examples further illustrate the present invention in detail but are not to be construed to limit the scope thereof.

Example 1

Measurement of methylene blue using modulated light

In this example, modulated light is used to excite a dye catalyst in the presence of luminol. The chemiluminescence signal is processed by a lock-in amplifier to quantify the dye catalyst.

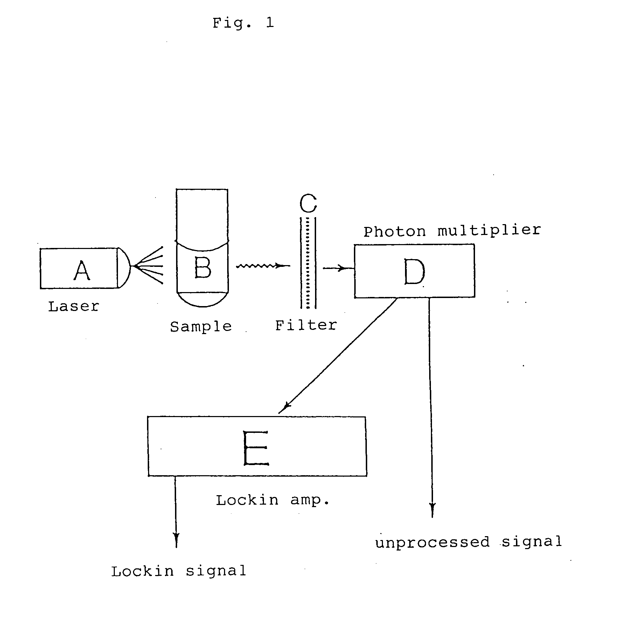

Chemical reaction solution (0.05M NaOH, 1mM luminol recrystallized from aqueous alkaline solution, 20% ethanol) is prepared. Methylene blue is dissolved in 100% ethanol and serial dilutions are made in ethanol. Each 10 $\mu$l is added to 1 ml chemical reaction solutions in 12mm by 75mm borosilicate glass sample test tubes to make final 100 fold dilutions. Each tube is placed into the chemiluminescence machine as shown in Fig. 1 to measure chemiluminescence.

The chemiluminescence machine to be used in the present invention is summarized in Fig. 1. A 670 nm 5 mW photodiode laser A (Toshiba Electric Co., Ltd., Japan; TOLD9211) controlled by a PNP transister in the grounded emitter mode irradiates sample tube B which is partially exposed to room light. The laser is turned on and off by a 500 Hz signal generator. The light emitted from sample tube B is filtered by 425 nm filter C (Kenko Hoya Co., Ltd., Japan; glass type B430). Filtered light enters photon multiplier D (Hamamatsu Photonics Co., Ltd., Japan; type R376). The photon derived voltage from D is locked in to the 500 Hz signal generator and processed by lock-in amplifier E (NF Circuit Design Block Co., Ltd., Japan; Model LI-574A).

For a control, the same samples and conditions were used with a photon counting photon multiplier (Hamamatsu Photonics co., Ltd., Japan; H-3460-04), a prescaler (Hamamatsu Photonics Co., Ltd., Japan; C-3589) and a frequency counter (IWATSU Universal Counter SC-7204).

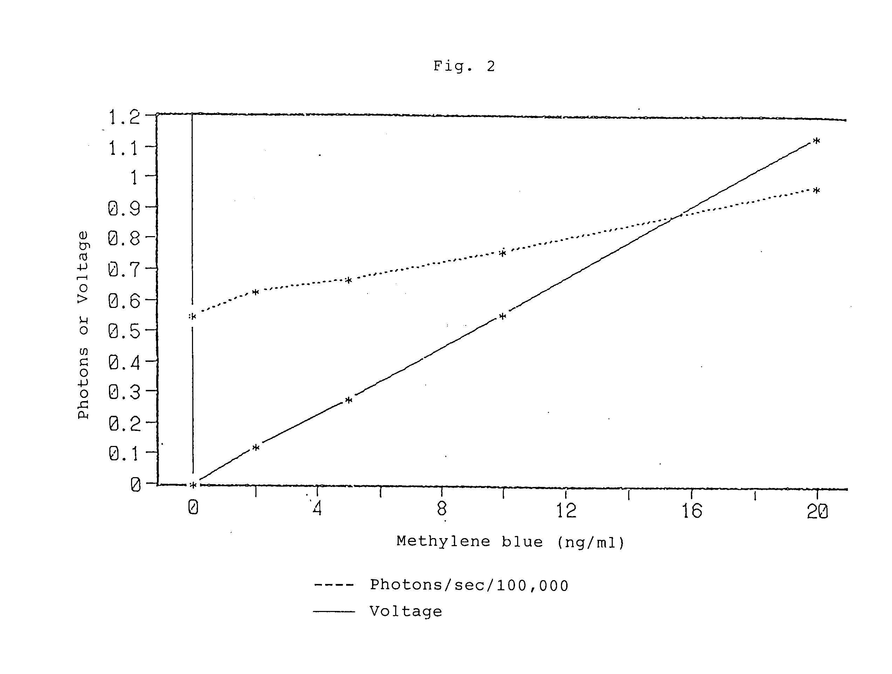

The standard curve obtained is shown in Fig. 2. Both the lock-in signal amplifier processed result and the control (unprocessed) result are shown in Fig. 2. As is seen from Fig. 2, the control measurements are less sensitive than the lock-in amplifier measurements.

Example 2

Pulse gated detection of the chemiluminescence reaction

In this example, a pulsating 670 nm light source is combined with time gating of photon counting to eliminate a background signal due to light scattering.

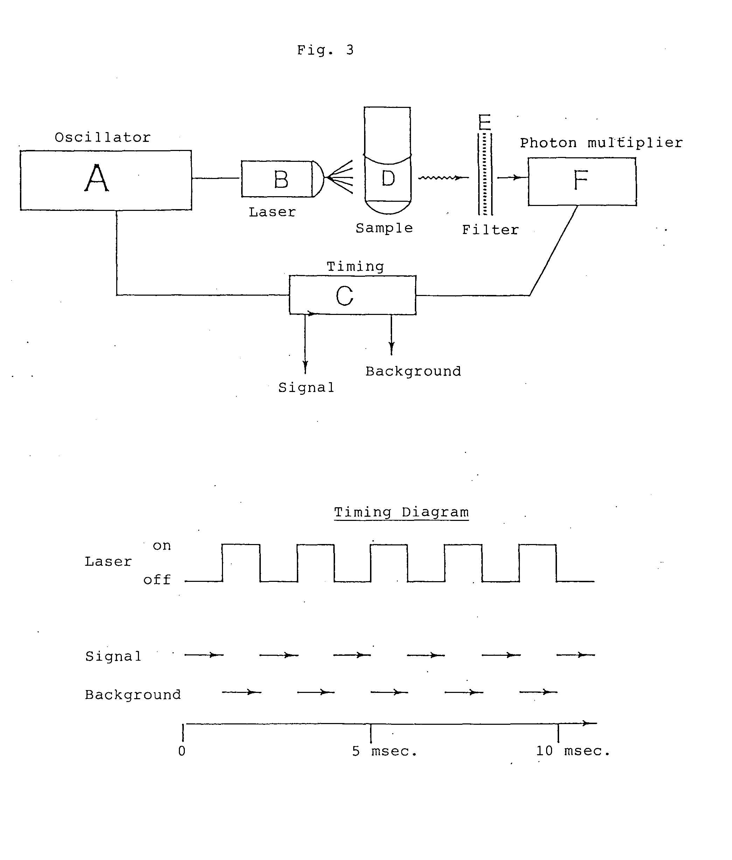

The chemiluminescence machine used in the present invention is summarized in Fig. 3. This contains a photon counter (Hamamatsu Photonics Co., Ltd., Japan; H-3460-04) and timing circuitry C which allows photon counting when the laser B (Toshiba Electric Co., Ltd., Japan; TOLD 9211) is off and separate counting when it is on. The laser B is turned on and off at a rate of 500Hz by a square wave signal. Oscillator A (explained above in Example 1) controls laser B and timing circuitry C. Laser B irradiates sample D with a periodic pulse as shown in the timing diagram. Light from sample D is filtered by 425 nm filter E (Kenko Hoya Co., Ltd., Japan; glass type B430) and enters photonmultiplier F. The signal from F is counted during two timing intervals as shown in the timing diagram. Light pulses counted in both intervals are separately integrated from 1 to 120 seconds after inserting a sample tube containing 1 ml of the sample by two frequency counters (IWATSU Universal Counter SC-7204).

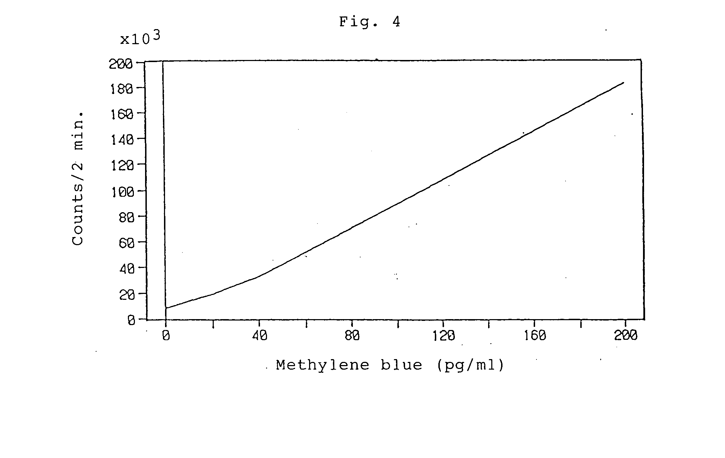

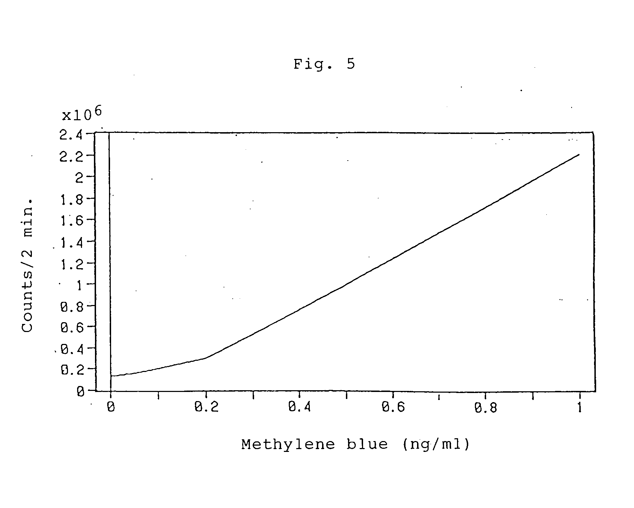

Methylene blue standards are prepared in 1.0 mM luminol, 0.05 M NaOH and 20% ethanol as described in Example 1. The methylene blue standard curve produced from the (laser off) signal is shown in Fig. 4. The detection limit from this data is 13 pg. When the total light emitted (signal plus background) is integrated for the methylene blue measurement, the sensitivity is poorer (see Fig. 5). The detection limit in the latter case is 220 pg.

## Claims

1. A method for determination of a light sensitive substance comprising irradiating the light sensitive substance in the presence of a lumigenic substance with modulated light and synchronously detecting shorter wavelength emitted light proportional to the concentration of the light sensitive substance.

2. A method according to claim 1, wherein the light sensitive substance is a member selected from the group consisting of xanthines, eosins, porphyrins, chlorins and thionines.

3. A method according to claim 1, wherein the lumigenic substance is a member selected from the group consisting of luminols, lucigenins and luciferins.

4. A method according to claim 1, wherein a laser or light emitting diode is used as the light source.

5. A method according to claim 1, wherein a lock-in amplifier is used for detection.

6. A method according to claim 1, wherein the light sensitive substance is conjugated to an antibody and participates in an immunoassay detection system.

7. A method for determination of a light sensitive substance comprising pulse-irradiating the light sensitive substance in the presence of a lumigenic substance and detecting shorter wavelength emitted light in the absence of the pulsed excitation light proportional to the concentration of the light sensitive substance.

8. A method according to claim 7, wherein the light sensitive substance is a member selected from the group consisting of xanthines, eosins, porphyrins, chlorins and thionines.

9. A method according to claim 7, wherein the lumigenic substance is a member selected from the group consisting of luminols, lucigenins and luciferins.

10. A method according to claim 7, wherein a laser or light emitting diode is used as the light source.

11. A method according to claim 7, wherein the light sensitive substance is conjugated to an antibody and participates in an immunoassay detection system.

Fig. 1

Fig. 2

Photons or Voltage

Methylene blue (ng/ml)

---- Photons/sec/100,000

——— Voltage

Fig. 3

Timing Diagram

Fig. 4

Fig. 5